# EUROPEAN PATENT SPECIFICATION

(11) **EP 4 494 618 B1**
(45) Date of publication and mention of the grant of the patent: **11.03.2026**
(21) Application number: 23796210.5
(22) Date of filing: 19.04.2023
(51) Int. Cl.: A61F 13/15

(54) **METHOD AND APPARATUS FOR MANUFACTURING DISPOSABLE WEAR**
VERFAHREN UND VORRICHTUNG ZUR HERSTELLUNG VON WEGWERFKLEIDUNG
PROCÉDÉ ET APPAREIL DE FABRICATION DE VÊTEMENT JETABLE

(30) Priority: 27.04.2022 JP 2022073573
(43) Date of publication of application: 22.01.2025
(73) Proprietor: Zuiko Corporation, Ibaraki-shi, Osaka 567-0082 (JP)
(72) Inventor: YAMAUCHI Hirofumi, Ibaraki-shi, Osaka 567-0082 (JP)
(74) Representative: Hasegawa, Kan
(86) International application number: PCT/JP2023/015679
(87) International publication number: WO 2023/210468

(56) References cited:
- WO-A1-2010/089964
- WO-A1-2012/032732
- JP-A- 2007 117 226
- JP-A- 2014 188 108
- JP-A- 2014 188 108
- US-A1- 2014 318 695

## Description

### TECHNICAL FIELD

The present invention relates to a method and an apparatus for manufacturing a disposable wear, and more specifically, relates to a technique for manufacturing a substantially T-shaped disposable wear.

### BACKGROUND ART

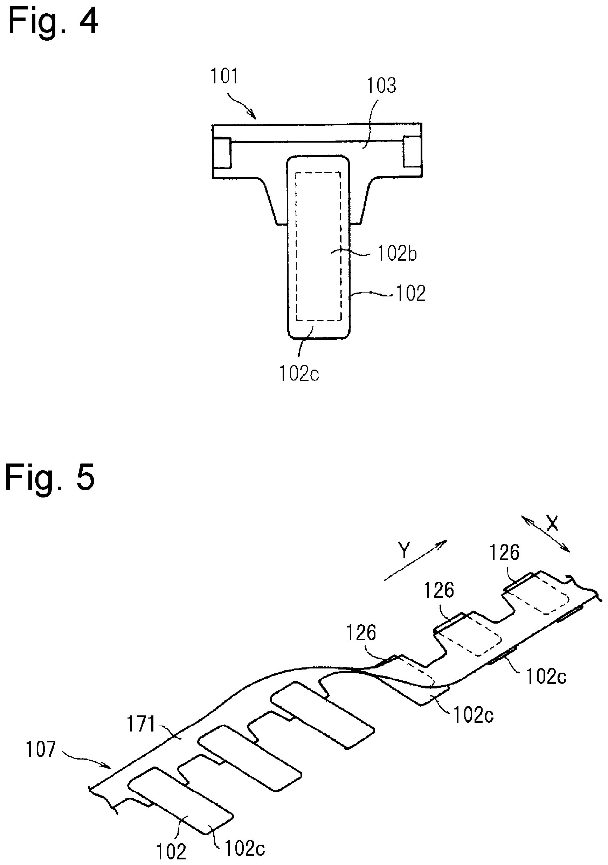

For example, FIG. 4 is a developed view of a substantially T-shaped disposable wear 101. As shown in FIG. 4, in the disposable wear 101, one end side of an absorbent body 102 is bonded to an intermediate position of a waist member 103. The disposable wear 101 is provided in a state where the other end side 102c of the absorbent body 102 is folded back at a central part 102b toward the waist member 103 so that the absorbent body 102 is folded into two parts and the waist member is folded so that both end parts overlap (see, for example, JP 4920424 B2).

JP 2014-188108 A discloses a method and apparatus for manufacturing disposable wear, comprising steps of cutting absorbent bodies from a continuous body, folding them into two parts, rotating them by 90 degrees, and bonding them to a waist-member continuous body.

FIG. 5 is a perspective view showing a manufacturing process of the disposable wear 101. As shown in FIG. **5****,** a wear continuous body 107 in which a plurality of absorbent bodies 102 have one end sides thereof bonded in a comb shape with a predetermined spacing therebetween is formed while a waist-member continuous body 171 in which portions to be made into waist members 103 are connected is conveyed in the direction indicated by the arrow Y. Then, while the wear continuous body 107 is being conveyed, the waist-member continuous body 171 side of the wear continuous body 107 is lifted and placed on the other end sides 102c of the absorbent bodies 102 to fold the absorbent bodies 102 into two parts so that fold lines 126 are formed at intermediate positions of the absorbent bodies 102 in a direction X orthogonal to the conveyance direction Y. Then, the wear continuous body 107 is cut between the adjoining absorbent bodies 102 to form disposable wears (see, for example, JP 4920424 B2).

### SUMMARY OF INVENTION

### PROBLEM TO BE SOLVED BY THE INVENTION

In a case where the absorbent bodies are folded into two parts while the wear continuous body is being conveyed as described above, the absorbent bodies 102 are folded into two parts with the angle being gradually increased so that no excessive force acts on the absorbent bodies 102. For this reason, the section where the absorbent bodies 102 are to be folded into two parts is long, which increases the installation space of the disposable wear manufacturing apparatus.

In view of such circumstances, a problem to be solved by the present invention is to provide a method and an apparatus for manufacturing a disposable wear with which it is possible to shorten the section where the absorbent bodies are to be folded into two parts.

### MEANS FOR SOLVING THE PROBLEM

The problem is solved by the teachings of the independent claims. Further embodiments are defined in the dependent claims. The invention is defined by the appended claims.

The present disclosure provides a disposable wear manufacturing method structured as follows:

The disposable wear manufacturing method is provided with: (i) a first conveying step of conveying, in a first direction, an absorbent-body continuous body in which first portions to be made into absorbent bodies are connected in the first direction; (ii) a second conveying step of conveying, in a second direction, a waist-member continuous body in which second portions to be made into waist members are connected in the second direction; (iii) a first cutting step of forming the absorbent bodies by cutting the absorbent-body continuous body being conveyed in the first direction; (iv) a folding step of forming folded absorbent bodies by folding the cut absorbent bodies into two parts so that fold lines are formed in a direction orthogonal to the first direction while conveying the cut absorbent bodies along a cylindrical conveyance path; (v) a rotating step of rotating the folded absorbent bodies by 90 degrees while conveying the folded absorbent bodies; (vi) a bonding step of forming a wear continuous body by placing more than one of the 90-degree rotated absorbent bodies on the waist-member continuous body being conveyed in the second direction, with a spacing therebetween, and bonding the absorbent bodies to the waist-member continuous body; and (vii) a second cutting step of forming disposable wears in each of which the folded absorbent body is bonded to an intermediate position of the waist member, by cutting the wear continuous body at cutting positions between the adjoining absorbent bodies.

According to the above-described method, since the absorbent bodies are folded into two parts while being conveyed along the cylindrical conveyance path, the section where the absorbent bodies are to be folded into two parts can be made short compared with when the absorbent bodies are folded into two parts while being conveyed along a linear conveyance path.

Preferably, the disposable wear manufacturing method is further provided with (viii) a temporarily fixing step of temporarily fixing together a first region and a second region divided by the fold line of each of the folded absorbent bodies.

In this case, the temporarily fixed folded absorbent bodies can be conveyed at higher speed because the folded state is stabilized, so that the number of disposable wears manufactured per unit time can be increased.

Preferably, the disposable wear manufacturing method is further provided with (ix) a first repitching step where by using a first moving pad, the folded absorbent bodies are received from the folding step, the received folded absorbent bodies are transferred to the rotating step, and the first moving pad receives the folded absorbent bodies from the folding step while moving at a first reception speed and transfers the received folded absorbent bodies to the rotating step while moving at a first transfer speed different from the first reception speed.

In this case, it is possible to accommodate size differences of the absorbent bodies.

Preferably, the disposable wear manufacturing method is further provided with (x) a second repitching step where by using a second moving pad, the 90-degree rotated folded absorbent bodies are received from the rotating step, the received folded absorbent bodies are transferred to the bonding step, and the second moving pad receives the 90-degree rotated folded absorbent bodies from the rotating step while moving at a second reception speed and transfers the received folded absorbent bodies to the bonding step while moving at a second transfer speed different from the second reception speed.

In this case, it is possible to accommodate size differences of the waist members.

Preferably, the disposable wear manufacturing method is further provided with: (ix) a first repitching step where by using a first moving pad, the folded absorbent bodies are received from the folding step, the received folded absorbent bodies are transferred to the rotating step, and the first moving pad receives the folded absorbent bodies from the folding step while moving at a first reception speed and transfers the received folded absorbent bodies to the rotating step while moving at a first transfer speed different from the first reception speed; and (x) a second repitching step where by using a second moving pad, the 90-degree rotated folded absorbent bodies are received from the rotating step, the received folded absorbent bodies are transferred to the bonding step, and the second moving pad receives the 90-degree rotated folded absorbent bodies from the rotating step while moving at a second reception speed and transfers the received folded absorbent bodies to the bonding step while moving at a second transfer speed different from the second reception speed.

In this case, even if the sizes of both of the absorbent bodies and the waist members change according to the size of the disposable wears, it is possible to accommodate size differences of the disposable wears.

Moreover, the present disclosure provides a disposable wear manufacturing apparatus structured as follows:

The disposable wear manufacturing apparatus is provided with: (a) a first unit that forms absorbent bodies by cutting an absorbent-body continuous body in which first portions to be made into the absorbent bodies are connected in a first direction, while conveying the absorbent-body continuous body in the first direction, and forms folded absorbent bodies by folding the absorbent bodies into two parts so that fold lines are formed in a direction orthogonal to a conveyance direction of the absorbent bodies, while conveying the absorbent bodies along a cylindrical conveyance path: (b) a rotating unit that rotates the folded absorbent bodies by 90 degrees while conveying the folded absorbent bodies: and (c) a second unit that forms a wear continuous body by placing more than one of the 90-degree rotated folded absorbent bodies on a waist member continuous body in which second portions to be made into waist members are connected in a second direction, with a spacing therebetween while conveying the waist member continuous body in the second direction and bonding the folded absorbent bodies to the waist-member continuous body, and forms disposable wears in each of which the folded absorbent body is bonded to an intermediate position of the waist member, by cutting the wear continuous body at cutting positions between the adjoining absorbent bodies.

According to the above-described structure, since the absorbent bodies are folded into two parts while being conveyed along the cylindrical conveyance path, the section where the absorbent bodies are to be folded into two parts can be made short compared with when the absorbent bodies are folded into two parts while being conveyed along a linear conveyance path.

Preferably, the disposable wear manufacturing apparatus is further provided with: (d) a temporarily fixing device that temporarily fixes together a first region and a second region divided by the fold line of each of the folded absorbent bodies.

In this case, the folded absorbent bodies can be conveyed at higher speed because the folded state of the absorbent bodies is stabilized, so that the number of disposable wears manufactured per unit time can be increased.

Preferably, the disposable wear manufacturing apparatus is further provided with: (e) a first repitching unit having a first moving pad that receives the folded absorbent bodies from the first unit while moving at a first reception speed and transfers the received folded absorbent bodies to the rotating unit while moving at a first transfer speed different from the first reception speed.

In this case, it is possible to accommodate size differences of the absorbent bodies.

Preferably, the disposable wear manufacturing apparatus is further provided with: (f) a second repitching unit having a second moving pad that receives the 90-degree rotated folded absorbent bodies from the rotating unit while moving at a second reception speed and transfers the received absorbent bodies to the second unit while moving at a second transfer speed different from the second reception speed.

In this case, it is possible to accommodate size differences of the waist members.

Preferably, the disposable wear manufacturing apparatus is further provided with: (g) a first repitching unit having a first moving pad that receives the folded absorbent bodies from the first unit while moving at a first reception speed and transfers the received folded absorbent bodies to the rotating unit while moving at a first transfer speed different from the first reception speed; and: (f) a second repitching unit having a second moving pad that receives the 90-degree rotated folded absorbent bodies from the rotating unit while moving at a second reception speed and transfers the received absorbent bodies to the second unit while moving at a second transfer speed different from the second reception speed.

In this case, even if the sizes of both of the absorbent bodies and the waist members change according to the size of the disposable wears, it is possible to accommodate size differences of the disposable wears.

### EFFECTS OF THE INVENTION

According to the present invention, it is possible to shorten the section where the absorbent bodies are to be folded into two parts.

### BRIEF DESCRIPTION OF THE DRAWINGS

[Fig. 1] FIG. 1 is a schematic view of a disposable wear manufacturing apparatus (first embodiment).
[Fig. 2] FIG. 2 is a schematic view of the disposable wear manufacturing apparatus (first embodiment).
[Fig. 3] FIG. 3 is an explanatory view of a disposable wear manufacturing process (first embodiment).
[Fig. 4] FIG. 4 is a developed view of the disposable wear (first conventional example).
[Fig. 5] FIG. 5 is a perspective view showing the manufacturing process of the disposable wear (first conventional example).

### MODE FOR CARRYING OUT THE INVENTION

Hereinafter, an embodiment of the present invention will be described with reference to the drawings.

### [First embodiment]

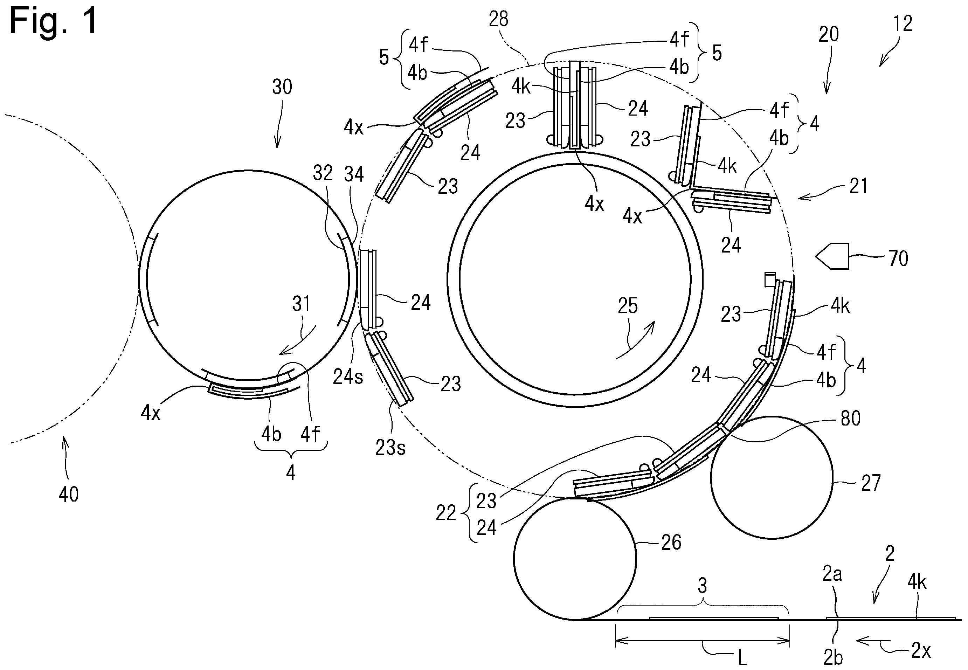

A disposable wear manufacturing method and apparatus of a first embodiment will be described with reference to FIGS. 1 to 3.

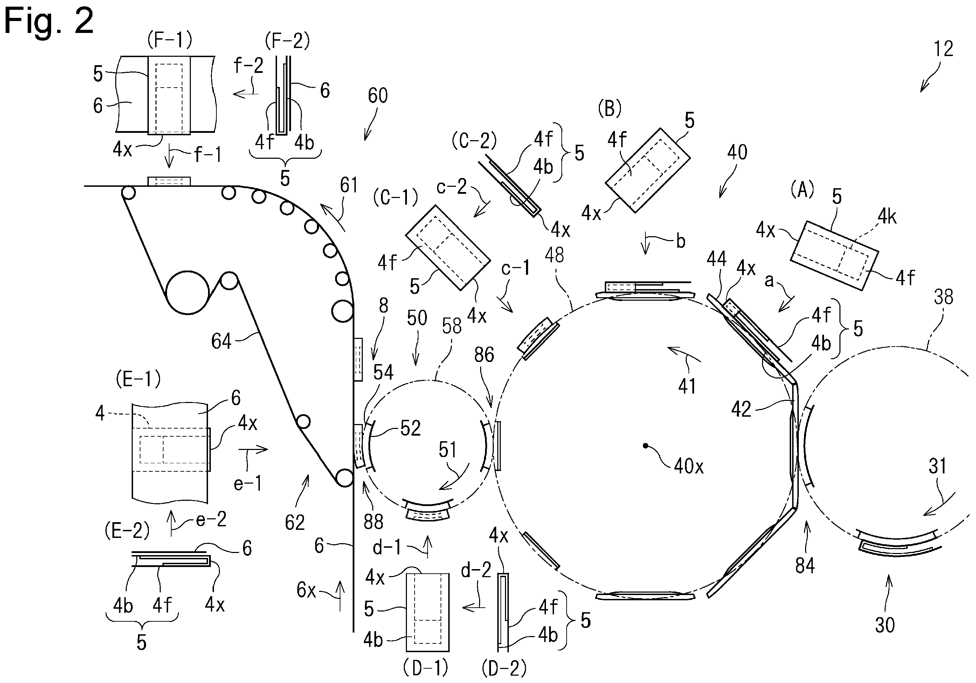

FIGS. 1 and 2 are schematic views of a disposable wear manufacturing apparatus 12. In FIG. 2, (A), (B), (C-1), (C-2), (D-1), (D-2), (E-1), (E-2), (F-1) and (F-2) are schematic views from the directions indicated by the arrows a, b, c-1, c-2, d-1, d-2, e-1, e-2, f-1 and f-2, respectively.

As shown in FIGS. 1 and 2, the disposable wear manufacturing apparatus 12 is provided with a first unit 20, a first repitching unit 30, a rotating unit 40, a second repitching unit 50, a second unit 60 and a temporarily fixing device 70.

In the first unit 20, a guide roll 26 and a cutter roll 27 are disposed adjacent to a folding drum 21. The folding drum 21 conveys an absorbent-body continuous body 2 fed through the guide roll 26, along a cylindrical conveyance path 28. The cutter roll 27 cuts the absorbent-body continuous body 2 being conveyed by the folding drum 21, thereby forming absorbent bodies 4. The folding drum 21 folds the absorbent bodies 4 into two parts while conveying them along the cylindrical conveyance path 28, thereby forming folded absorbent bodies 5.

In the absorbent-body continuous body 2, first portions 3 having a predetermined length L and to be made into the absorbent bodies 4 are connected in a first direction 2x indicated by the arrow 2x. For example, in the absorbent-body continuous body 2, absorbent cores 4k containing pulp and/or superabsorbent polymer or the like are arranged in the first direction 2x with a spacing therebetween between two belt-form sheets 2a and 2b. The parts between the adjoining absorbent cores 4k of the absorbent-body continuous body 2 are cut, whereby the absorbent bodies 4 are formed.

The folding drum 21 is structured similarly to the folding device disclosed in Japanese Patent No. 2851784. The folding drum 21 is provided with a plurality of suction units 22 that move in the direction indicated by the arrow 25 along the cylindrical conveyance path 28. The suction units 22 each have a pair of suction tables 23 and 24 that open and close while moving. The suction tables 23 and 24 have suction surfaces 23s and 24s. Although not shown, suction holes communicating with a negative pressure source are formed in the suction surfaces 23s and 24s. The absorbent-body continuous body 2 fed through the guide roll 26 is sucked to the suction surfaces 23s and 24s of the opened suction surfaces 23 and 24, and conveyed.

The cutter roll 27 cuts the absorbent-body continuous body 2 between the adjoining suction units 22 when the parts between the adjoining suction units 22 of the absorbent-body continuous body 2 pass a facing position 80 where the cutter roll 27 and the folding drum 21 face each other, thereby forming the absorbent bodies 4. A first region 4f of each of the absorbent bodies 4 on the front side in the conveyance direction is sucked to the suction surface 23s of preceding one suction table 23 of the suction unit 22, and a second region 4b of each of the absorbent bodies 4 on the back side in the conveyance direction is sucked to the suction surface 24s of the other suction table 24 of the suction unit 22.

While sucking and conveying the absorbent body 4, the suction units 22 each sandwich the absorbent body 4 between the suction tables 23 and 24 by the suction tables 23 and 24 closing so as to face each other. Thereby, the absorbent body 4 is folded into two parts so as to form a fold line 4x orthogonal to the conveyance direction of the absorbent body 4 in which the absorbent body 4 is conveyed by the suction unit 22, whereby the folded absorbent body 5 is formed. The folded absorbent body 5 is divided into the first region 4f and the second region 4b by the fold line 4x.

After forming the folded absorbent body 5, the suction units 22 each make one suction table 23 stop sucking so that only the other suction table 24 sucks the folded absorbent body 5, and returns to the state where the suction tables 23 and 24 are open while moving along the cylindrical conveyance path 28. Then, when the other suction table 24 passes the transfer position facing the first repitching unit 30, the other suction table 24 stops sucking to transfer the folded absorbent body 5 to the first repitching unit 30.

The first repitching unit 30 has a first moving pad 32 that moves along a cylindrical conveyance path 38 while changing the speed. The first moving pad 32 has a suction surface 34 where suction holes communicating with a non-illustrated negative pressure source are formed. While moving at a first reception speed, the first moving pad 32 sucks, to the suction surface 34, the folded absorbent body 5 sucked to the suction table 24 of the folding drum 21 of the first unit 20, receives the folded absorbent body 5 from the folding drum 21, and while moving at a first transfer speed different from the first reception speed, transfers the received folded absorbent body 5 to the rotating unit 40. Structuring the first moving pad 32 so as to be moved by using a servo motor makes it easy to change the movement speed of the first moving pad 32.

The rotating unit 40 has a rotating pad 42 that rotates 90 degrees while moving along a cylindrical conveyance path 48. The rotating pad 42 has a suction surface 44 where suction holes communicating with a non-illustrated negative pressure source are formed, and receives the folded absorbent body 5 from the first repitching unit 30 by sucking, to the suction surface 44, the folded absorbent body 5 sucked to the first moving pad 32 of the first repitching unit 30. At this time, the rotating pad 42 receives the folded absorbent body 5 so that the fold line 4x of the folded absorbent body 5 is at the front. While conveying the folded absorbent body 5 while sucking it to the suction surface 44, the rotating pad 42 makes the fold line 4x of the folded absorbent body 5 parallel to the conveyance direction of the folded absorbent body 5 by rotating the folded absorbent body 5 by 90 degrees about a rotation axis extending in the normal direction of the rotating unit 40, and transfers the folded absorbent body 5 to the second repitching unit 50.

The second repitching unit 50 has a second moving pad 52 that moves along a cylindrical conveyance path 58 while changing the speed, and the second moving pad 52 has a suction surface 54 where suction holes communicating with a non-illustrated negative pressure source are formed. While moving at a second reception speed, the second moving pad 52 receives, from the rotating unit 40, the folded absorbent body 5 sucked to the rotating pad 42 of the rotating unit 40 and rotated by 90 degrees, by sucking the 90-degree rotated folded absorbent body 5 to the suction surface 54, and while moving at a second transfer speed different from the second reception speed, transfers the received folded absorbent body 5 to the second unit 60. Structuring the second moving pad 52 so as to be moved by a servo motor makes it easy to change the movement speed of the second moving pad 52.

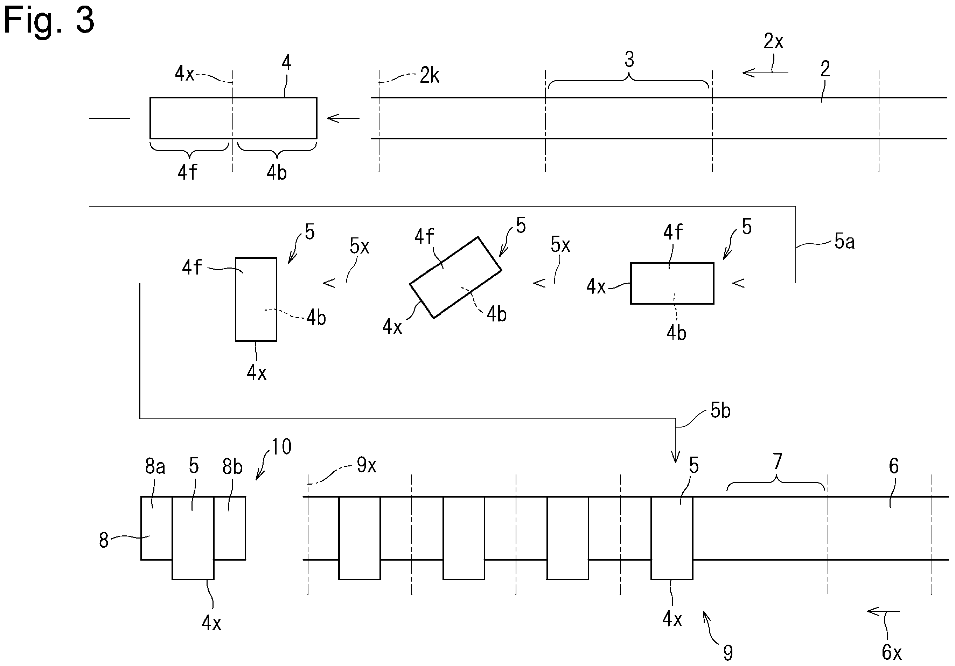

The second unit 60 has a conveyer 62 that conveys a waist-member continuous body 6, and an adhesive applier and a cutter which are not shown. In the waist-member continuous body 6, second portions 7 (see FIG. 3) to be made into waist members 8 (see FIG. 3) are connected in a second direction 6x.

The conveyer 62 is provided with an endless belt 64 having air permeability and circulating along a predetermined path and a non-illustrated suction box disposed inside the endless belt 64 and connected to a negative pressure source, and is structured so as to convey the waist-member continuous body 6 while sucking it to the outside of the endless belt 64.

The conveyer 62 is disposed adjacent to the second repitching unit 50, and is structured so that the 90-degree rotated folded absorbent bodies 5 from the second repitching unit 50 are placed on the waist-member continuous body 6 being conveyed by the conveyer 62, with a spacing therebetween.

The non-illustrated applier previously applies an adhesive to one or both of the overlapping parts of the waist-member continuous body 6 and the 90-degree rotated folded absorbent bodies 5.

When the 90-degree rotated folded absorbent bodies 5 are placed on the waist-member continuous body 6, they are bonded together to form a wear continuous body 9. The wear continuous body 9 is conveyed by the conveyer 62. The folded absorbent bodies 5 are bonded to the waist-member continuous body 6 in a state of being placed on the waist-member continuous body 6 so as to be along the waist-member continuous body 6 with the fold line 4x protruding from the waist-member continuous body 6. Instead of using an adhesive, the folded absorbent bodies 5 and the waist-member continuous body 6 may be bonded together by heat sealing, ultrasonic wave sealing or the like with the folded absorbent bodies 5 being placed on the waist-member continuous body 6.

The non-illustrated cutter cuts the wear continuous body 9 at cutting positions 9x (see FIG. 3) between the adjoining folded absorbent bodies 5 of the wear continuous body 9, thereby forming disposable wears 10 (see FIG. 3) where the folded absorbent body 5 is bonded at an intermediate position of the waist member 8 (see FIG. 3).

The temporarily fixing device 70 temporarily fixes together the first region 4f and the second region 4b divided by the fold line 4x of each folded absorbent body 5. That is, the bonding between the first region 4f and the second region 4b is made with a strength that can be released when the disposable wear 10 is used.

The temporarily fixing device 70 applies an adhesive to appropriate positions of the absorbent bodies 4 before being folded into two parts. The temporarily fixing device 70 may be structured so as to apply double-sided tape to appropriate positions of the absorbent bodies 4 before being folded into two parts or may be structured so as to temporarily fix appropriate positions of the folded absorbent bodies 5 after being folded into two parts, by bonding by heat sealing or ultrasonic wave sealing.

While the temporarily fixing device 70 may be omitted, performing temporarily fixing with the temporarily fixing device 70 being provided stabilizes the folded state and makes it possible to convey the folded absorbent bodies 5 at higher speed, so that the number of disposable wears 10 manufactured per unit time can be increased.

While the first repitching unit 30 may be omitted, the provision of the first repitching unit 30 between the first unit 20 and the rotating unit 40 makes it possible to accommodate size differences of the absorbent bodies 4.

For example, in a case where the size of the absorbent bodies 4 in the first direction 2x, that is, the predetermined length L is different, to manufacture the disposable wears 10 in the same cycle time, that is, to make the same the number of disposable wears 10 manufactured per unit time, if the speed at which the folded absorbent bodies 5 are conveyed at the rotating unit 40 is made the same, it is necessary to change the speed at which the absorbent-body continuous body 2 and the absorbent bodies 4 are conveyed at the first unit 20. Moreover, even if the size of the absorbent bodies 4 in the first direction 2x (predetermined length L) is the same, when the size of the absorbent bodies 4 in the width direction orthogonal to the first direction 2x is different, to manufacture the disposable wears 10 in the same cycle time, if the speed at which the first unit 20 conveys the absorbent-body continuous body 2 and the absorbent bodies 4 is made the same, it is necessary to change the speed at which the folded absorbent bodies 5 are conveyed at the rotating unit 40. As described above, when the conveyance speed of the folded absorbent bodies 5 at the first unit 20 and the conveyance speed of the folded absorbent bodies 5 at the rotating unit 40 are not the same, the first repitching unit 30 can stably convey the folded absorbent bodies 5 from the first unit 20 to the rotating unit 40. Consequently, the size differences of the absorbent bodies 4 can be accommodated.

Moreover, the provision of the first repitching unit 30 between the first unit 20 and the rotating unit 40 makes it possible to perform the folding of the absorbent bodies 4 and the rotating of the folded absorbent body 5 in an upward-facing state where bonding is more stabilized. This allows the absorbent bodies 4 and the folded absorbent bodies 5 to be conveyed at higher speed, which enables more efficient manufacture of the disposable wears 10.

While the second repitching unit 50 may be omitted, the provision of the second repitching unit 50 between the rotating unit 40 and the second unit 60 makes it possible to accommodate size differences of the waist members 8.

For example, when the size of the waist members 8 in the second direction 6x is different, to manufacture the disposable wears 10 in the same cycle time, if the conveyance speed of the 90-degree rotated folded absorbent bodies 5 at the rotating unit 40 is maintained the same, it is necessary to change the conveyance speed of the waist-member continuous body 6 at the second unit 60. In such a case, the second repitching unit 50 can change the conveyance speed of the 90-degree rotated folded absorbent bodies 5 when they are placed on the waist-member continuous body 6, so as to coincide with the conveyance speed of the waist-member continuous body 6. This makes it possible to accommodate size differences of the waist members.

The provision of both of the first repitching unit 30 and the second repitching unit 50 makes it possible to accommodate size differences of the disposable wears 10 even if the sizes of both of the absorbent bodies 4 and the waist members 8 change according to the size of the disposable wears 10.

In the disposable wear manufacturing apparatus 12, since the folding drum 21 of the first unit 20 folds the absorbent bodies 4 into two parts while conveying them along the cylindrical conveyance path 28, the section where the absorbent bodies 4 are to be folded into two parts can be made short compared with when the absorbent bodies 4 are folded into two parts while being conveyed along a linear conveyance path.

Next, a method for manufacturing the disposable wear 10 will be described with reference to FIG. 3. FIG. 3 is an explanatory view showing a disposable wear manufacturing process. As shown in FIG. 3, the disposable wear 10 is formed by using the absorbent-body continuous body 2 and the waist-member continuous body 6.

The method for manufacturing the disposable wear 10 is provided with the following steps (1) to (7):

### (1) First conveying step

The absorbent-body continuous body 2 is conveyed in the first direction 2x indicated by the arrow 2x. In the absorbent-body continuous body 2, the first portions 3 to be made into the absorbent bodies 4 are connected in the first direction 2x. The absorbent bodies 4 may be continuously formed and conveyed, or previously prepared absorbent bodies 4 may be conveyed.

### (2) Second conveying step

The waist-member continuous body 6 is conveyed in the second direction 6x indicated by the arrow 6x. In the waist-member continuous body 6, the second portions 7 to be made into the waist members 8 are connected in the second direction 6x. The waist-member continuous body 6 may be continuously formed and conveyed, or a previously prepared waist-member continuous body 6 may be conveyed.

### (3) First cutting step

The absorbent-body continuous body 2 being conveyed in the first direction 2x is cut at the cutting positions indicated by a chain line 2k, thereby forming the absorbent bodies 4.

### (4) Folding step

As shown by the arrow 5a, while being conveyed in the cylindrical conveyance path 28 (see FIG. 1), the cut absorbent bodies 4 are each folded into two parts at an intermediate position of the absorbent body 4 in the first direction 2x so that the fold line 4x orthogonal to the first direction 2x is formed, thereby forming the folded absorbent bodies 5. The folded absorbent bodies 5 are each divided into the first region 4f and the second region 4b by the fold line 4x, and the first region 4f and the second region 4b overlap each other.

### (5) Rotating step

The folded absorbent bodies 5 are rotated by 90 degrees while being conveyed in the direction indicated by the arrow 5x. That is, the direction of the fold line 4x of each folded absorbent body 5 relative to the conveyance direction 5x is changed by 90 degrees so that the conveyance direction 5x of the folded absorbent bodies 5 and the fold line 4x are parallel to each other.

### (6) Bonding step

As shown by the arrow 5b, the 90-degree rotated folded absorbent bodies 5 are placed on the waist-member continuous body 6 being conveyed in the second direction 6x, and bonded thereto. That is, a plurality of 90-degree rotated folded absorbent bodies 5 are placed on the waist-member continuous body 6 being conveyed in the second direction 6x, with a spacing therebetween, and the folded absorbent bodies 5 are bonded to the waist-member continuous body 6 to form the wear continuous body 9. At this time, the folded absorbent bodies 5 are placed on the waist-member continuous body 6 so as to be along the waist-member continuous body 6 with the fold lines 4x of the folded absorbent bodies 5 protruding from the waist-member continuous body 6, and the part of each folded absorbent body 5 facing the waist-member continuous body 6 is bonded to the waist-member continuous body 6. For example, the folded absorbent bodies 5 and the waist-member continuous body 6 are bonded together by previously applying an adhesive to appropriate positions of the folded absorbent bodies 5 and/or the waist-member continuous body 6 or by heat sealing, ultrasonic wave sealing or the like with the folded absorbent bodies 5 being placed on the waist-member continuous body 6.

### (7) Second cutting step

The wear continuous body 9 is cut at the cutting positions 9x indicated by the chain line 9x between the adjoining folded absorbent bodies 5, thereby forming the disposable wears 10 where the folded absorbent body 5 is bonded to an intermediate position of the waist member 8.

To the waist member 8, one of the first and second regions 4f and 4b of the folded absorbent body 5 is bonded. A non-illustrated hook-and-loop fastener for bonding to the other of the first and second regions 4f and 4b of the folded absorbent body 5 may be provided on parts 8a and 8b near both ends of the waist member 8. This hook-and-loop fastener may be previously provided on the waist-member continuous body 6, or may be provided on the disposable wears 10 after the cutting. Other accessory members may be previously provided on the absorbent-body continuous body 2 and/or the waist-member continuous body 6, or may be provided on the disposable wears 10 after the cutting.

When the disposable wears 10 are formed by the above-described steps (1) to (7), since the absorbent bodies 4 are folded into two parts while being conveyed along the cylindrical conveyance path, the section where the absorbent bodies 4 are to be folded into two parts can be made short compared with when the absorbent bodies 4 are folded into two parts while being conveyed along a linear conveyance path.

The method for manufacturing the disposable wear 10 may be further provided with any one or more of the following steps (8) to (10).

### (8) Temporarily fixing step

The first region 4f and the second region 4b of the folded absorbent body 5 are temporarily fixed together. For example, they are temporarily fixed by applying an adhesive or applying double-sided tape to appropriate positions of the absorbent body 4 before folded into two parts. The temporary fixing may be performed by temporarily fixing appropriate positions of the folded absorbent body 5 by heat sealing, ultrasonic wave sealing or the like after the absorbent body 4 is folded into two parts. The folded absorbent body 5 having undergone the temporary fixing can be conveyed at higher speed because its folded state is stabilized, so that the number of disposable wears manufactured per unit time can be increased.

### (9) First repitching step

By using the first moving pad 32 (see FIG. 1), the folded absorbent bodies 5 are received from the folding step, and the received folded absorbent bodies 5 are transferred to the rotating step. The first moving pad 32 receives the folded absorbent bodies 5 from the folding step while moving at the first reception speed, and transfers the received folded absorbent bodies 5 to the rotating step while moving at the first transfer speed different from the first reception speed. The first repitching step makes it possible to accommodate size differences of the absorbent bodies.

### (10) Second repitching step

By using the second moving pad 52 (see FIG. 2), the 90-degree rotated folded absorbent bodies 5 are received from the rotating step, and the received folded absorbent bodies 5 are transferred to the bonding step. The second moving pad 52 receives the 90-degree rotated folded absorbent bodies 5 from the rotating step while moving at the second reception speed, and transfers the received folded absorbent bodies 5 to the bonding step while moving at the second transfer speed different from the second reception speed. The second repitching step makes it possible to accommodate size difference of the waist members.

The provision of both of the first repitching step and the second repitching step makes it possible to accommodate size differences of the disposable wears 10 even if the sizes of both of the absorbent bodies 4 and the waist members 8 change according to the size of the disposable wears 10.

### [Summary]

As described above, by folding the absorbent bodies into two parts while conveying them along the cylindrical conveyance path, the section where the absorbent bodies are to be folded into two parts can be shortened.

The present invention is not limited to the above-described embodiment but may be carried out with various modifications being added.

### DESCRIPTION OF REFERENCE NUMERALS

2 Absorbent-body continuous body
2x First direction
3 First portion
4 Absorbent body
4b Second region
4f First region
4x Fold line
5 Folded absorbent body
6 Waist-member continuous body
6x Second direction
7 Second portion
8 Waist member
9 Wear continuous body
9x Cutting position
10 Disposable wear
12 Disposable wear manufacturing apparatus
20 First unit
28 Cylindrical conveyance path
30 First repitching unit
32 First moving pad
40 Rotating unit
50 Second repitching unit
52 Second moving pad
60 Second unit
70 Temporarily fixing device

## Claims

1. A disposable wear manufacturing method comprising:
a first conveying step of conveying, in a first direction (2x), an absorbent-body continuous body (2) in which first portions (3) to be made into absorbent bodies (4) are connected in the first direction (2x);
a second conveying step of conveying, in a second direction (6x), a waist-member continuous body (6) in which second portions (7) to be made into waist members (8) are connected in the second direction (6x);
a first cutting step of forming the absorbent bodies (4) by cutting the absorbent-body continuous body (2) being conveyed in the first direction (2x);
a folding step of forming folded absorbent bodies (5) by folding the cut absorbent bodies (4) into two parts so that fold lines (4x) are formed in a direction orthogonal to the first direction (2x) while conveying the cut absorbent bodies (4);
a rotating step of rotating the folded absorbent bodies (5) by 90 degrees while conveying the folded absorbent bodies (5);
a bonding step of forming a wear continuous body (9) by placing more than one of the 90-degree rotated absorbent bodies (5) on the waist-member continuous body (6) being conveyed in the second direction (6x), with a spacing therebetween, and bonding the absorbent bodies (5) to the waist-member continuous body (6); and
a second cutting step of forming disposable wears (10) in each of which the folded absorbent body (5) is bonded to an intermediate position of the waist member (8), by cutting the wear continuous body (9) at cutting positions (9x) between the adjoining absorbent bodies (5),
**characterized in that**
at the first cutting step, the absorbent-body continuous body (2) is cut while conveying the absorbent-body continuous body (2) in the first direction (2x) along a cylindrical conveyance path (28), and
at the folding step, the cut absorbent bodies (4) are folded into two parts while conveying the cut absorbent bodies (4) along the cylindrical conveyance path (28).

2. The disposable wear manufacturing method according to claim 1, further comprising
a temporarily fixing step of temporarily fixing together a first region (4f) and a second region (4b) divided by the fold line (4x) of each of the folded absorbent bodies (5).

3. The disposable wear manufacturing method according to claim 1 or 2, further comprising
a first repitching step where by using a first moving pad (32), the folded absorbent bodies (5) are received from the folding step, the received folded absorbent bodies (5) are transferred to the rotating step, and the first moving pad (32) receives the folded absorbent bodies (5) from the folding step while moving at a first reception speed and transfers the received folded absorbent bodies (5) to the rotating step while moving at a first transfer speed different from the first reception speed.

4. The disposable wear manufacturing method according to claim 1 or 2, further comprising
a second repitching step where by using a second moving pad (52), the 90-degree rotated folded absorbent bodies (5) are received from the rotating step, the received folded absorbent bodies (5) are transferred to the bonding step, and the second moving pad (52) receives the 90-degree rotated folded absorbent bodies (5) from the rotating step while moving at a second reception speed and transfers the received folded absorbent bodies (5) to the bonding step while moving at a second transfer speed different from the second reception speed.

5. The disposable wear manufacturing method according to claim 1 or 2, further comprising:
a first repitching step where by using a first moving pad (32), the folded absorbent bodies (5) are received from the folding step, the received folded absorbent bodies (5) are transferred to the rotating step, and the first moving pad (32) receives the folded absorbent bodies (5) from the folding step while moving at a first reception speed and transfers the received folded absorbent bodies (5) to the rotating step while moving at a first transfer speed different from the first reception speed; and
a second repitching step where by using a second moving pad (52), the 90-degree rotated folded absorbent bodies (5) are received from the rotating step, the received folded absorbent bodies (5) are transferred to the bonding step, and the second moving pad (52) receives the 90-degree rotated folded absorbent bodies (5) from the rotating step while moving at a second reception speed and transfers the received folded absorbent bodies (5) to the bonding step while moving at a second transfer speed different from the second reception speed.

6. A disposable wear manufacturing apparatus (12) comprising:
a first unit (20) that forms absorbent bodies (4) by cutting an absorbent-body continuous body (2) in which first portions (3) to be made into the absorbent bodies (4) are connected in a first direction (2x), while conveying the absorbent-body continuous body (2) in the first direction (2x), and forms folded absorbent bodies (5) by folding the absorbent bodies (4) into two parts so that fold lines (4x) are formed in a direction orthogonal to a conveyance direction of the absorbent bodies (4);
a rotating unit (40) that rotates the folded absorbent bodies (5) by 90 degrees while conveying the folded absorbent bodies (5); and
a second unit (60) that forms a wear continuous body (9) by placing more than one of the 90-degree rotated folded absorbent bodies (5) on a waist member continuous body (6) in which second portions (7) to be made into waist members (8) are connected in a second direction (6x), with a spacing therebetween while conveying the waist member continuous body (6) in the second direction (6x) and bonding the folded absorbent bodies (5) to the waist-member continuous body (6), and forms disposable wears (10) in each of which the folded absorbent body (5) is bonded to an intermediate position of the waist member (8), by cutting the wear continuous body (9) at cutting positions (9x) between the adjoining absorbent bodies (5),
charaterized in that
the first unit (20) cuts the absorbent-body continuous body (2) while conveying the absorbent-body continuous body (2) in the first direction (2x) along a cylindrical conveyance path (28), and forms the folded absorbent bodies (5) while conveying the absorbent bodies (4) along the cylindrical conveyance path (28).

7. The disposable wear manufacturing apparatus (12) according to claim 6, further comprising
a temporarily fixing device (70) that temporarily fixes together a first region (4f) and a second region (4b) divided by the fold line (4x) of each of the folded absorbent bodies (5).

8. The disposable wear manufacturing apparatus (12) according to claim 6 or 7, further comprising
a first repitching unit (30) having a first moving pad (32) that receives the folded absorbent bodies (5) from the first unit (20) while moving at a first reception speed and transfers the received folded absorbent bodies (5) to the rotating unit (40) while moving at a first transfer speed different from the first reception speed.

9. The disposable wear manufacturing apparatus (12) according to claim 6 or 7, further comprising
a second repitching unit (50) having a second moving pad (52) that receives the 90-degree rotated folded absorbent bodies (5) from the rotating unit (40) while moving at a second reception speed and transfers the received absorbent bodies (5) to the second unit (60) while moving at a second transfer speed different from the second reception speed.

10. The disposable wear manufacturing apparatus (12) according to claim 6 or 7, further comprising:
a first repitching unit (30) having a first moving pad (32) that receives the folded absorbent bodies (5) from the first unit (20) while moving at a first reception speed and transfers the received folded absorbent bodies (5) to the rotating unit (40) while moving at a first transfer speed different from the first reception speed; and
a second repitching unit (50) having a second moving pad (52) that receives the 90-degree rotated folded absorbent bodies (5) from the rotating unit (40) while moving at a second reception speed and transfers the received absorbent bodies (5) to the second unit (60) while moving at a second transfer speed different from the second reception speed.

## Patentansprüche

1. Ein Herstellungsverfahren für Einwegkleidung, das umfasst:
ein erster Übertragungsschritt ist in der ersten Richtung (2x) ein saugkörper-kontinuierlicher Körper (2), bei dem die ersten Teile (3), die zu absorbierenden Körpern (4) gemacht werden sollen, in der ersten Richtung verbunden werden (2x);
ein zweiter Übertragungsschritt, der in einer zweiten Richtung (6x) einen durchgehenden Körper an den Taillenteilen (6) überführt, bei dem die zweiten Teile (7), die zu Taillenteilen (8) gemacht werden sollen, in der zweiten Richtung verbunden sind (6x);
ein erster Schneideschritt: die Absorptionskörper (4) durch das Schneiden des kontinuierlichen Körpers des Absorptionskörpers (2), der in die erste Richtung transportiert wird (2x);
ein Faltschritt, bei dem gefaltete Absorptionskörper (5) gefaltet werden, indem die geschnittenen Absorptionskörper (4) in zwei Teile gefaltet werden, sodass Faltlinien (4x) in einer Richtung geformt werden, die orthogonal zur ersten Richtung (2x) ist, während die Schnittabsorberkörper übertragen werden (4);
ein Drehschritt, bei dem die gefalteten Absorptionskörper (5) um 90 Grad gedreht werden, während die gefalteten Absorptionskörper transportiert werden (5);
ein Bindungsschritt, bei dem ein durchgehender Körper (9) gebildet wird, indem mehr als einer der um 90 Grad gedrehten Absorptionskörper (5) auf den durchgehenden Körper des Taillenteils (6), der in die zweite Richtung transportiert wird (6x), mit einem Abstand dazwischen, und das Verbinden der absorbierenden Körper (5) mit dem kontinuierlichen Körper des Taillenteils (6); und
Ein zweiter Schneideschritt, die Einwegverschleiße (10) formt, bei denen der gefaltete Saugkörper (5) an eine Zwischenposition des Taillenteils (8) gebunden wird, indem der durchgehende Verschleißkörper (9) an Schneidpositionen (9x) zwischen den angrenzenden Saugkörpern (5) durchtrennt wird.
charakterisiert darin, dass
Beim ersten Schneideschritt wird der saugfähige Körper (2) geschnitten, während der saugfähige Körper (2) in der ersten Richtung (2x) entlang eines zylindrischen Transportwegs (28) transportiert wird, und
Im Faltschritt werden die geschnittenen Absorptionskörper (4) in zwei Teile gefoldet, während die geschnittenen Absorptionskörper (4) entlang des zylindrischen Transportwegs transportiert werden (28).

2. Das Herstellungsverfahren für Einwegkleidung gemäß Anspruch 1, das im Folgenden umfasst
ein temporärer Fixierungsschritt, bei dem ein erster Bereich (4f) und ein zweiter Bereich (4b) durch die Faltungslinie (4x) jedes der gefalteten Absorptionskörper (5) geteilt werden.

3. Das Herstellungsverfahren für Einwegkleidung gemäß Anspruch 1 oder 2, das weitere umfasst
Ein erster Repitching-Schritt, bei dem mit einem ersten bewegten Pad (32) die gefalteten Absorptionskörper (5) vom Faltschritt empfangen werden, die empfangenen gefalteten Absorptionskörper (5) auf den Rotationsschritt übertragen werden und das erste bewegte Pad (32) die gefalteten Absorptionskörper (5) vom Faltschritt empfängt, während es sich mit der ersten Empfangsgeschwindigkeit bewegt und die empfangenen gefalteten Absorptionskörper (5) in den Rotationsschritt überträgt, während es sich mit einer ersten Bewegung bewegt Die Übertragungsgeschwindigkeit unterscheidet sich von der ersten Empfangsgeschwindigkeit.

4. Das Herstellungsverfahren für Einwegverkleidung gemäß Anspruch 1 oder 2, das im Folgenden umfasst
Ein zweiter Repitching-Schritt, bei dem mit einem zweiten beweglichen Pad (52) die um 90 Grad gefalteten Absorptionskörper (5) vom Drehschritt empfangen werden, die empfangenen gefalteten Absorptionskörper (5) in den Bonding-Schritt übertragen werden und das zweite Moving Pad (52) die um 90 Grad gefalteten Absorptionskörper (5) aus dem Rotationsschritt empfängt, während es sich mit einer zweiten Empfangsgeschwindigkeit bewegt und die empfangenen gefalteten Absorptionskörper (5) auf die Bindung überträgt Schritt, während sie sich mit einer zweiten Übertragungsgeschwindigkeit bewegt, die sich von der zweiten Empfangsgeschwindigkeit unterscheidet.

5. Das Herstellungsverfahren für Einwegverkleidung gemäß Anspruch 1 oder 2, das weiter:
Ein erster Repitching-Schritt, bei dem mit einem ersten bewegten Pad (32) die gefalteten Absorptionskörper (5) vom Faltschritt empfangen werden, die empfangenen gefalteten Absorptionskörper (5) auf den Rotationsschritt übertragen werden und das erste bewegte Pad (32) die gefalteten Absorptionskörper (5) vom Faltschritt empfängt, während es sich mit der ersten Empfangsgeschwindigkeit bewegt und die empfangenen gefalteten Absorptionskörper (5) in den Rotationsschritt überträgt, während es sich mit einer ersten Bewegung bewegt die Übertragungsgeschwindigkeit, die sich von der ersten Empfangsgeschwindigkeit unterscheidet; und
Ein zweiter Repitching-Schritt, bei dem mit einem zweiten beweglichen Pad (52) die um 90 Grad gefalteten Absorptionskörper (5) vom Drehschritt empfangen werden, die empfangenen gefalteten Absorptionskörper (5) in den Bonding-Schritt übertragen werden und das zweite Moving Pad (52) die um 90 Grad gefalteten Absorptionskörper (5) aus dem Rotationsschritt empfängt, während es sich mit einer zweiten Empfangsgeschwindigkeit bewegt und die empfangenen gefalteten Absorptionskörper (5) auf die Bindung überträgt Schritt, während sie sich mit einer zweiten Übertragungsgeschwindigkeit bewegt, die sich von der zweiten Empfangsgeschwindigkeit unterscheidet.

6. Eine Einweg-Kleidungs-Herstellungsanlage (12) umfasst:
Eine erste Einheit (20), die Absorptionskörper (4) bildet, indem sie einen Absorptionskörper kontinuierlichen Körper (2) schneidet, wobei die ersten Teile (3), die zu den Absorptionskörpern (4) gemacht werden, in einer ersten Richtung verbunden sind (2x), während der Absorptionskörper kontinuierlicher Körper (2) in die erste Richtung (2x) transportiert wird, und bildet gefaltete Absorptionskörper (5), indem die absorbierenden Körper (4) in zwei Teile gefaltet werden, sodass Faltlinien (4x) in einer Richtung gebildet werden, die orthogonal zu ist eine Transportrichtung der Absorptionskörper (4);
eine rotierende Einheit (40), die die gefalteten Saugkörper (5) um 90 Grad dreht, während sie die gefalteten Absorptionskörper transportiert (5); und
eine zweite Einheit (60), die einen durchgehenden Körper (9) bildet, indem mehr als einer der um 90 Grad gefalteten Saugkörper (5) auf einen durchgehenden Körper des Taillenteils (6) gesetzt wird, wobei die zweiten Teile (7) zu Taillenteilen (8) in eine zweite Richtung (6x) verbunden sind, mit einem Abstand dazwischen, während der fortlaufende Körper des Taillenteils (6) in die zweite Richtung (6x) übertragen und die gefalteten absorbierenden Körper verbunden werden (5) an den durchgehenden Körper des Taillenteils (6) und bildet Einwegverkleidungen (10), bei denen jeweils der gefaltete saugfähige Körper (5) an eine Zwischenposition des Taillenteils (8) gebunden wird, indem der durchgehende Körper (9) an den Schneidpositionen (9x) zwischen den angrenzenden saugfähigen Körpern (5) durchtrennt wird,
charakterisiert darin, dass
Die erste Einheit (20) schneidet den saugfähigen Körper (2) durch, während sie den absorbierenden Körper (2) in der ersten Richtung (2x) entlang eines zylindrischen Transportwegs (28) transportiert und die gefalteten Absorptionskörper (5) bildet, während sie die absorbierenden Körper (4) entlang des zylindrischen Transportwegs (28) transportiert.

7. Die Einweg-Kleidungs-Herstellungsapparatur (12) gemäß Anspruch 6, die weiter umfasst
ein temporäres Fixierungsgerät (70), das vorübergehend einen ersten Bereich (4f) und einen zweiten Bereich (4b) zusammenhält, geteilt durch die Faltlinie (4x) jedes der gefalteten Absorptionskörper (5).

8. Die Einweg-Kleidungs-Herstellungsapparatur (12) gemäß Anspruch 6 oder 7, die weiter umfasst
eine erste Wiederaufnahmeeinheit (30) hat ein First Moving Pad (32), das die gefalteten Absorptionskörper (5) von der ersten Einheit (20) empfängt, während sie sich mit der ersten Empfangsgeschwindigkeit bewegt und die empfangenen gefalteten Absorptionskörper (5) auf die rotierende Einheit (40) überträgt, während sie sich mit einer ersten Übertragungsgeschwindigkeit unterscheidet.

9. Das Einweg-Kleidungs-Herstellungsapparat (12) gemäß Anspruch 6 oder 7, der weiter umfasst
eine zweite Repitching-Einheit (50) mit einer zweiten beweglichen Platte (52), die die um 90 Grad gefalteten Absorptionskörper (5) von der rotierenden Einheit (40) empfängt, während sie sich mit einer zweiten Empfangsgeschwindigkeit bewegt und die empfangenen Absorptionskörper (5) auf die zweite Einheit (60) überträgt, während sie sich mit einer zweiten Übertragungsgeschwindigkeit unterscheidet.

10. Das Einweg-Kleidungs-Produktionsapparat (12) gemäß Behauptung 6 oder 7, die weiter:
eine erste Umstellungseinheit (30) mit einer zuerst bewegenden Platte (32), die die gefalteten Absorptionskörper (5) von der ersten Einheit (20) empfängt, während sie sich mit der ersten Empfangsgeschwindigkeit bewegt und die empfangenen gefalteten Absorptionskörper (5) auf die rotierende Einheit (40) überträgt, während sie sich mit einer ersten Übertragungsgeschwindigkeit unterscheidet; und
eine zweite Repitching-Einheit (50) mit einer zweiten beweglichen Platte (52), die die um 90 Grad gefalteten Absorptionskörper (5) von der rotierenden Einheit (40) empfängt, während sie sich mit einer zweiten Empfangsgeschwindigkeit bewegt und die empfangenen Absorptionskörper (5) auf die zweite Einheit (60) überträgt, während sie sich mit einer zweiten Übertragungsgeschwindigkeit unterscheidet.

## Revendications

1. Une méthode de fabrication de l'usure jetable comprenant :
une première étape de transmission consiste à transporter, dans une première direction (2x), un corps continu à corps absorbant (2) dans lequel les premières parties (3) à transformer en corps absorbants (4) sont reliées dans la première direction (2x) ;
une seconde étape de transmission consistant à transporter, dans une seconde direction (6x), un corps continu de taille (6) dans lequel les secondes parties (7) à transformer en éléments de taille (8) sont reliées dans la seconde direction (6x) ;
une première étape de coupe consiste à former les corps absorbants (4) en coupant le corps continu absorbant (2) étant conduit dans la première direction (2x) ;
une étape de pliage consistant à former les corps absorbants pliés (5) en pliant les corps absorbants découpés (4) en deux parties de sorte que les lignes de pliage (4x) soient formées dans une direction orthogonale à la première direction (2x) tout en transportant les corps absorbants coupés (4) ;
une étape rotative consistant à faire pivoter les corps absorbants pliés (5) de 90 degrés tout en transportant les corps absorbants repliés (5) ;
une étape de collage consistant à former un corps continu d'usure (9) en plaçant plus d'un des corps absorbants rotatifs à 90 degrés (5) sur le corps continu du membre de taille (6) en étant transporté dans la seconde direction (6x), avec un espacement entre les deux, et en liant les corps absorbants (5) au corps continu du membre de taille (6) ; et
une seconde étape de coupe consiste à former des usures jetables (10) dans chacune d'elles le corps absorbant plié (5) étant fixé à une position intermédiaire du membre de taille (8), en coupant le corps continu d'usure (9) aux positions de coupe (9x) entre les corps absorbants adjacents (5),
**caractérisé par** cela
À la première étape de coupe, le corps continu à corps absorbant (2) est coupé tout en transportant le corps continu à corps absorbant (2) dans la première direction (2x) le long d'un chemin de transport cylindrique (28), et
À l'étape de pliage, les corps absorbants coupés (4) sont foledés en deux parties pendant que les corps absorbants coupés (4) sont transportés le long du chemin de transport cylindrique (28).

2. La méthode de fabrication des vêtements jetables selon la revendication 1, comprenant en outre
une étape temporaire de fixation consistant à fixer temporairement ensemble une première région (4f) et une seconde région (4b) divisée par la ligne de pli (4x) de chacun des corps absorbants repliés (5).

3. La méthode de fabrication des vêtements jetables selon la revendication 1 ou 2, comprenant en outre
une première étape de repitching où, en utilisant un premier pad mobile (32), les corps absorbants repliés (5) sont reçus de l'étape de pliage, les corps absorbants repliés reçus (5) sont transférés à l'étape rotatif, et le premier pad mobile (32) reçoit les corps absorbants repliés (5) de l'étape de pliage en se déplaçant à une vitesse de première réception et transfère les corps absorbants repliés reçus (5) à l'étape rotative tout en se déplaçant à une première Vitesse de transfert différente de la première réception.

4. La méthode de fabrication des vêtements jetables selon la revendication 1 ou 2, comprenant en outre
une seconde étape de repitching où, en utilisant une seconde plaque mobile (52), les corps absorbants pliés et rotés à 90 degrés (5) sont reçus de l'étape de rotation, les corps absorbants repliés reçus (5) sont transférés à l'étape de liaison, et la seconde plateforme mobile (52) reçoit les corps absorbants pliés et pliés à 90 degrés (5) de l'étape en rotation tout en se déplaçant à une seconde vitesse de réception et transfère les corps absorbants repliés reçus (5) à la liaison Pas en se déplaçant à une vitesse de transfert différente de la vitesse de réception seconde.

5. La méthode de fabrication de l'usure jetable selon la revendication 1 ou 2, comprenant en outre :
une première étape de repitching où, en utilisant un premier pad mobile (32), les corps absorbants repliés (5) sont reçus de l'étape de pliage, les corps absorbants repliés reçus (5) sont transférés à l'étape rotatif, et le premier pad mobile (32) reçoit les corps absorbants repliés (5) de l'étape de pliage en se déplaçant à une vitesse de première réception et transfère les corps absorbants repliés reçus (5) à l'étape rotative tout en se déplaçant à une première vitesse de transfert différente de la première vitesse de réception ; et
une seconde étape de repitching où, en utilisant une seconde plaque mobile (52), les corps absorbants pliés et rotés à 90 degrés (5) sont reçus de l'étape de rotation, les corps absorbants repliés reçus (5) sont transférés à l'étape de liaison, et la seconde plateforme mobile (52) reçoit les corps absorbants pliés et pliés à 90 degrés (5) de l'étape en rotation tout en se déplaçant à une seconde vitesse de réception et transfère les corps absorbants repliés reçus (5) à la liaison Pas en se déplaçant à une vitesse de transfert différente de la vitesse de réception seconde.

6. Un appareil de fabrication de vêtements jetables (12) comprenant :
une première unité (20) qui forme les corps absorbants (4) en découpant un corps continu de corps absorbant (2) dans lequel les premières parties (3) à former dans les corps absorbants (4) sont reliées dans une première direction (2x), tout en transportant le corps continu du corps absorbant (2) dans la première direction (2x), et forme les corps absorbants repliés (5) en repliant les corps absorbants (4) en deux parties de sorte que les lignes de plissement (4x) soient formées dans une direction orthogonale à une direction de transport des corps absorbants (4) ;
une unité rotative (40) qui fait tourner les corps absorbants pliés (5) de 90 degrés tout en transportant les corps absorbants pliés (5) ; et
une seconde unité (60) qui forme un corps continu d'usure (9) en plaçant plus d'un des corps absorbants pliés et pliés à 90 degrés (5) sur un corps continu de la taille (6) dans lequel les secondes parties (7) à transformer en éléments de taille (8) sont reliées dans une seconde direction (6x), avec un espacement entre les deux, tout en transportant le corps continu du membre de la taille (6) dans la seconde direction (6x) et en liant les corps absorbants pliés (5) au corps continu du membre de taille (6), et forme des usures jetables (10) dans chacune d'elles, le corps absorbant replié (5) est lié à une position intermédiaire du membre de taille (8), en coupant le corps continu d'usure (9) aux positions de coupe (9x) entre les corps absorbants adjacents (5),
**caractérisé par** cela
La première unité (20) coupe le corps continu du corps absorbant (2) tout en transportant le corps continu du corps absorbant (2) dans la première direction (2x) le long d'un chemin de transport cylindrique (28), et forme les corps absorbants repliés (5) tout en transportant les corps absorbants (4) le long du chemin de transport cylindrique (28).

7. L'appareil de fabrication des vêtements jetables (12) selon la revendication 6, comprenant en outre
un dispositif temporairement fixant (70) qui fixe temporairement ensemble une première région (4f) et une seconde région (4b) divisées par la ligne de pliement (4x) de chacun des corps absorbants repliés (5).

8. L'appareil de fabrication des vêtements jetables (12) selon la revendication 6 ou 7, comprenant en outre
Une première unité de remise (30) possède un premier pad mobile (32) qui reçoit les corps absorbants repliés (5) de la première unité (20) tout en se déplaçant à une vitesse de première réception et transfère les corps absorbants repliés reçus (5) vers l'unité rotative (40) tout en se déplaçant à une vitesse de premier transfert différente de la première vitesse de réception.

9. L'appareil de fabrication des vêtements jetables (12) selon la revendication 6 ou 7, comprenant en outre
une seconde unité de repitching (50) possède un second pad mobile (52) qui reçoit les corps absorbants repliés et rotés à 90 degrés (5) de l'unité rotative (40) tout en se déplaçant à une seconde vitesse de réception et transfère les corps absorbants reçus (5) à la seconde unité (60) tout en se déplaçant à une seconde vitesse de transfert différente de la seconde vitesse de réception.

10. L'appareil de fabrication d'usure jetable (12) selon la revendication 6 ou 7, comprenant en outre :
une première unité de repitching (30) ayant un premier pad mobile (32) qui reçoit les corps absorbants repliés (5) de la première unité (20) tout en se déplaçant à une vitesse de première réception et transfère les corps absorbants repliés reçus (5) à l'unité rotative (40) tout en se déplaçant à une vitesse de premier transfert différente de la première vitesse de réception ; et
une seconde unité de repitching (50) possède un second pad mobile (52) qui reçoit les corps absorbants repliés et rotés à 90 degrés (5) de l'unité rotative (40) tout en se déplaçant à une seconde vitesse de réception et transfère les corps absorbants reçus (5) à la seconde unité (60) tout en se déplaçant à une seconde vitesse de transfert différente de la seconde vitesse de réception.
